# EUROPEAN PATENT APPLICATION

(11) **EP 0 834 293 A1**
(43) Date of publication of application: **08.04.1998**
(21) Application number: 97203012.6
(22) Date of filing: 01.10.1997
(51) Int. Cl.: A61F 2/06, A61M 25/10

(54) **Balloon catheter for placing a stent**

(30) Priority: 01.10.1996 NL 1004162
(71) Applicant: Cordis Europa N.V., 9302 LJ Roden (NL)
(72) Inventor: Almeleh, Raphael, 1180 Brussels (BE); Jansen, Wilhelmus Adrianus Petrus Cornelis, 9302 GC Roden (NL)
(74) Representative: 't Jong, Bastiaan Jacobus

(57) **Abstract**

This invention relates to a catheter comprising a tube-like basic body with a proximal and a distal end, at least one balloon member arranged close to the distal end, which can be expanded from a deflated state in which the balloon member has a small diameter to an inflated state in which the balloon member has a larger diameter. The catheter also comprises means for supplying a fluid under pressure to the balloon member in order to expand the latter, wherein the balloon member has, at least in the deflated state, a surface structure incorporating differences in height.

## Description

The invention relates to a balloon catheter particularly designed for the purpose of placing a stent inside a patient. In this case stent is understood to mean any tubular prosthesis used to support the wall of a body vessel of a patient.

Balloon catheters used for the purpose of introducing a stent are generally known. The stent is clasped around the balloon member in compressed state and is manoeuvred by means of the catheter into the target position inside the body of the patient. In that position the balloon is expanded from the deflated state into an inflated state by supplying a medium under pressure to the balloon member. Due to this expansion the stent is expanded at the same time, until it comes into contact with the wall of the body vessel. After the balloon member is restored to the deflated state again, the catheter can be removed and the stent remains behind.

It does happen that when introducing the catheter, the stent is displaced on the balloon member. When this happens the treatment is to be stopped and a new catheter with stent has to be introduced. In the worst case it may even happen that the stent comes loose from the catheter, which is obviously highly undesirable.

The object of the invention is to provide a balloon catheter of the type described in the preamble, which can be used to place a stent in a reliable manner in its position.

This aim is achieved with the balloon catheter according to the invention with the measure as characterised in claim 1. The surface structure of the balloon member ensures that the balloon member has in many places a good grip on the stent introduced by it.

In case the stent introduced with the balloon catheter according to the invention has a smooth inner surface, the surface structure of the balloon member will ensure an improved grip on the edges of the stent.

The invention relates to and provides however also a balloon catheter whereby the balloon member supports a non-expanded stent which has an internal surface with a surface structure incorporating differences in height with which the surface structure of the balloon member engages. Thus a maximum grip of the stent on the balloon member is effected.

The surface structure of the balloon member may have been achieved in many different ways. Preferably the measure as set out in claim 4 is employed. The protruding surface parts may be formed in a manner known as such, whereby the balloon member is expanded in a mould of which the mould cavity has a surface structure corresponding to the required surface structure. The surface structure is impressed on the balloon member. Thus a surface structure is created which has evenly distributed, protruding surface parts.

Further characteristics of preferred embodiments of the invention will become evident from the following description with reference to the attached drawings, in which the invention is explained in greater detail.

Figure 1 shows a perspective view of a balloon catheter according to the invention with a stent arranged around it.

Figure 2 shows a detailed view of the section indicated by arrow II in figure 1.

The figures 3 and 4 illustrate variants of embodiments of the balloon member of the catheter according to the invention.

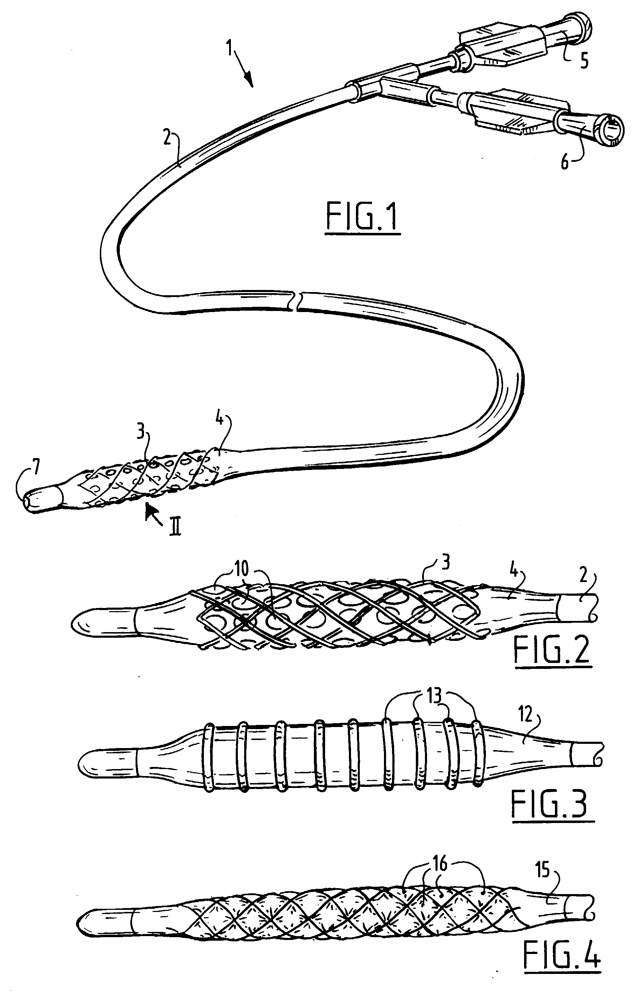

The catheter 1 illustrated in figure 1 comprises a tube-like basic body 2, to the distal end of which, as seen in the figure on the left, a balloon member has been arranged.

At the opposite proximal end of the catheter 1 connecting members 5, 6 have been arranged. A lumen 7 has been formed inside the basic body 2 which is accessible via the connecting member 5 and may be used for instance for receiving a guide wire.

The connecting member is connected with the inside of the balloon member 4 via a lumen not illustrated her, in order to be able to supply a medium under pressure to the balloon member in question in order to be able to expand the latter from a deflated state into an inflated state.

Around the balloon member 4 a stent 3 has been arranged. This stent has been arranged around the deflated balloon member 4 in a compressed state. In this state the stent 3 can be introduced into a patient by means of the catheter 1.

When the catheter 1 has been manoeuvred inside the body of the patient in such a position that the balloon member 4 and the stent 3 arranged around it are positioned at the site where the stent 3 has to be placed, for instance inside a blood vessel in an area where the vascular wall is impaired, fluid under pressure is supplied via the connecting member 6 to the balloon member 4 in order to expand the latter into the inflated state, expanding at the same time the stent 3 until it comes into contact with the wall of the vessel.

The stent 3 has in the usual manner been made in such a way that it maintains the expanded state and does not resume its original state after restoring the balloon member 4 to its deflated state again. As a result the balloon member 4 can be withdrawn from the expanded stent 3 and the catheter removed from the body of the patient, leaving the stent in situ.

It will be clear that when introducing the catheter, shearing forces will act on the stent 3 which could result in movement of the stent 3 on the balloon member 4. When passing the catheter introduction sheath for instance, these shearing forces may be considerable.

According to the invention, the balloon member 4 has been provided with a surface structure incorporating differences in height which are formed in the case of this example of an embodiment by evenly distributed dot-shaped protruding surface parts 10. In the example of this embodiment the stent 3 has been manufactured of a number of metal wires so that this stent has an internal surface with a surface structure containing differences in height with which the protruding surface parts 10 can engage. At all the places where a wire of the stent 3 makes contact with a protruding dot 10, a longitudinal force of the balloon member 4 can be applied to the stent 3. The totality of all those applied longitudinal forces is more than sufficient to ensure that the stent 3 is taken along with the balloon member 4 in a reliable manner when introducing the catheter.

The dot-shaped protruding surface parts 10 may have been preformed in the balloon member by heat treatment. In that case the heated balloon 4 is received in a mould with a mould cavity in which corresponding indentations have been formed in the wall. By expanding the balloon member 4 it will make contact with the wall surface of the mould cavity and will adopt the surface structure of the mould cavity. After the balloon member has cooled down, this surface structure has been 'frozen' into the balloon member.

After arranging the stent 3 in the compressed state to it, a fluid under a limited pressure may be conveyed, when employing the catheter according to the invention, via the connecting member 6 to the balloon member 4 in order to ensure that the protruding surface parts actually protrude and engage with the stent 3.

Figure 3 illustrates another embodiment of the catheter according to the invention. The balloon member 12 in this embodiment has been provided with protruding surface parts in the shape of rings 13. This shape is particularly suitable for use with a stent which has a smooth internal surface. The rings 13 of the surface structure which are located outside the stent arranged around the balloon 12 engage with the edges of the stent. Consequently a considerable longitudinal force may be applied to the stent as a result of which the stent will be taken along with the catheter in a reliable manner and cannot move in relation to the latter.

With the embodiment of figure 4 a wafer-shaped surface structure has been formed. The wafers 16 form protruding surface parts which can engage with a stent, or of which the rims can engage with the edges of the stent respectively.

The balloon of the catheter according to the invention may have been made of such a material that the surface structure is smoothed out and disappears in the inflated state. It is important that the surface structure is present in the deflated state or the slightly inflated state, the state of the balloon member when the stent 3 is advanced to its position.

## Claims

1. Catheter comprising a tube-like basic body with a proximal and a distal end, at least one balloon member arranged close to the distal end, which can be expanded from a deflated state in which the balloon member has a small diameter to an inflated state in which the balloon member has a larger diameter and means for supplying a fluid under pressure to the balloon member in order to expand the latter, wherein the balloon member has, at least in the deflated state, a surface structure incorporating differences in height.

2. Catheter as claimed in claim 1, wherein the balloon member carries a non-expanded stent.

3. Catheter as claimed in claim 2, wherein the stent has an internal surface with a surface structure with differences in height with which the surface structure of the balloon member engages.

4. Catheter as claimed in claim 1, wherein the surface structure has been formed by evenly distributed protruding surface parts.

5. Catheter as claimed in claim 4, wherein the protruding surface parts are rings.

6. Catheter as claimed in claim 4, wherein the protruding surface parts are a helically-shaped ridge.

7. Catheter as claimed in claim 4, wherein the protruding surface parts form a wafer-pattern.
